# NEW EUROPEAN PATENT SPECIFICATION

(11) **EP 3 313 372 B2**
(45) Date of publication and mention of the opposition decision: **26.11.2025**
(45) Mention of the grant of the patent: 29.04.2020
(21) Application number: 16813361.9
(22) Date of filing: 30.05.2016
(51) Int. Cl.: A61F 6/00, A61F 6/04, B65B 25/14, B65B 51/22, B65B 51/14, B65B 55/22, B65B 5/04

(54) **PACKAGED SILICONE LUBRICATED CONDOM THAT PROVIDES SENSATION**
VERPACKTES SILIKONGESCHMIERTES KONDOM MIT VERMITTLUNG EINES GEFÜHLS
PRÉSERVATIF LUBRIFIÉ AU SILICONE EMBALLÉ QUI PROCURE UNE SENSATION

(30) Priority: 24.06.2015 US 201562183844 P
(43) Date of publication of application: 02.05.2018
(73) Proprietor: LifeStyles Healthcare Pte. Ltd., Singapore 049712 (SG)
(72) Inventor: NGUYEN, Kc, Dothan, AL 36303 (US); CHUAH, Beng, Sim, Petaling Jaya 47301, Selangor (MY); TAN, Sek, Yan, Seri Kembangan 43300 Selangor (MY); CHOOI, Ji, An, Bayan Lepas 11900 Penang (MY); NGOWPRASERT, Chayapon, Surat Thani 84130 (TH); PONGTHANOMSAK, Chayaporn, Surat Thani 84130 (TH); TANJAROON, Phuttatida, Surat Thani 84130 (TH); MOHAMAD TAHIR, Hanita, Binti, Melaka 75450 (MY)
(74) Representative: Maiwald GmbH
(86) International application number: PCT/AU2016/000183
(87) International publication number: WO 2016/205853

(56) References cited:
- WO-A1-2014/055621
- WO-A1-2014/055621
- WO-A1-2014/055627
- WO-A1-2014/152154
- JP-A- 2013 138 754
- US-A1- 2003 215 532
- US-A1- 2006 034 936
- US-A1- 2006 240 997

## Description

The present application relates generally to packaged silicone lubricated condoms that in use provide sensation, such as warming, cooling or tingling.

Product information on the internet indicates that Trojan Tingly Warmth contains dimethicone, dimethiconol, hexyl nicotinate and vanillyl butyl ether. Dimethiconol is a small hydroxylated analogy of silicone oil, and is believed to be a stronger solvating agent for compounds with polar groups.

Product information on the internet for Durex Play Tingle is in conflict. The Durex.com (Ask Durex, FAQ) website states: "Sensilube, Play 2-in-1 Massage, Play Warming and Play Classic are water-based and Play Tingle is silicone-based, so all are totally safe to use with latex and non-latex condoms." On the other hand, the Product Data Sheet for Australia and New Zealand states that all the Durex Play Personal Lubricants, including Play Tingle personal lube are water-based. Yet, at the Amazon website it is said to have a "Long lasting silicone formula." At www.durex.co.uk/durex-play-tingle-lube-60ml it is said to not contain menthol and to be "water soluble and easily washed off." Given the emphasis on water solubility, it cannot be silicone oil based.

Silicone lubricants are desirable for use with elastomeric condoms as they are highly compatible with the long term integrity of the condoms. Thus, it is highly desirable to use silicone lubricants with the sensation-providing agents in a condom. Yet the sensation-providing agents vanillyl butyl ether, menthyl lactate, menthol and combination thereof in silicone oil have not been used on a synthetic polyisoprene condom before as this is a first of its technology platform. These agents can be applied to the synthetic polyisoprene condom to provide the sensation and more sensitivity during coitus.

Moreover, it has now been found that as formulated for cooling, warming or tingling on a synthetic polyisoprene condom the lubricant provides one or more of intense, early onset, sustained sensation, while minimizing the number of users that experience irritation.
WO 2014/055621 A1 discloses a non-irritating personal lubricant composition comprising a silicone fluid carrier and a sensorial agent, the composition having a viscosity of at least 175 centistokes, the composition to be used with a condom.

### SUMMARY

The present invention relates to a packaged condom as set out in claim 1 and to a method of packaging a condom as set out in claim 7. In embodiments, if the lubricant comprises vanillyl butyl ether but neither menthyl lactate nor menthol in effective amounts, then (a) the solvent consists essentially of non-water soluble silicone oil, and (b) the lubricant is essentially free of compounds that enhance the solubility of vanillyl butyl ether in the non-water soluble silicone oil.

### DESCRIPTION OF THE DRAWINGS

So that the manner in which the above recited features of the present invention can be understood in detail, a more particular description of the invention, briefly summarized above, may be had by reference to embodiments, some of which are illustrated in the appended drawings. It is to be noted, however, that the appended drawings illustrate only illustrative embodiments of this invention and are therefore not to be considered limiting of its scope, for the invention may admit to other equally effective embodiments.
FIG. 1 shows a packaged condom 20 with a cover layer 12; and
FIG. 2 shows in concept a condom 2 to which has been applied lubricant 4 (shown for illustration before contacting the condom); the condom 2 is packaged between covers 12A and 12B, which have been joined at seal 14.

To facilitate understanding, identical reference numerals have been used, where possible, to designate comparable elements that are common to the figures. The figures are not drawn to scale and may be simplified for clarity. It is contemplated that elements and features of one embodiment may be beneficially incorporated in other embodiments without further recitation.

### DETAILED DESCRIPTION

Silicone oil as the main lubricant carrier is useful as its safety with latex film is well established. Not only that, silicone is known to be very inactive and, as used here, it does not inhibit any of the sensation-providing agents in delivering its respective sensation to the consumer upon contact. The silicone oil maintains good burst properties for the condom, without creating any discoloration to the condom polymer.

For a warming lubricant, illustrative formulations (by wt.) can be:

| **Ingredient** | (about) |
|---|---|
| Vanillyl Butyl Ether | 0.03 to 0.20 g |
| Dimethicone | to 100 g |

To prepare the lubricant, for example, measure the right amount of vanillyl butyl ether in the empty beaker and then add in silicone oil. Stir with moderate speed until the mixture is homogenous.

For the warming variant, vanillyl butyl ether ("VBE") is the sensation-providing agent that imparts the warming sensation and vasodilation upon application. This active also acts a flavoring agent, where it gives the consumer a tinge of vanilla flavor.

For a cooling lubricant, illustrative formulations (by wt.) can be:

| **Ingredient** | (about) |
|---|---|
| Menthyl Lactate | 0.10 to 0.50 g |
| Menthol (crystal) | 0.03 to 0.25 g |
| Dimethicone | to 100 g |

To prepare the lubricant, for example, measure the right amount of menthyl lactate and menthol crystal (grind to smaller pieces for easy dissolving) in the empty beaker. Add in silicone oil. Stir with moderate speed until the mixture is homogenous.

In embodiments of the two sensation-providing agents menthyl lactate and menthol in a cooling lubricant it can be important to control the ratio of menthol to menthyl lactate to achieve the desired immediate then sustained effect, without creating an irritating sensation. For example, menthol can comprises about 30% to about 45% (wt.), such as about 35% to about 45%, of the total of the two agents.

For the cooling variant, a unique combination of two actives is used to give a long lasting cooling effect. Menthol, a derivative of peppermint, gives an immediate cooling and refreshing sensation to consumer while Menthyl Lactate gives off a milder and long lasting cooling sensation with slight minty taste. This mixture provides a cooling effect which is immediate upon application and yet long lasting. In addition to that, the combination can be at amounts that ensures the cooling sensation is not too strong to be mistaken as irritant by the consumer

For a tingling lubricant, illustrative formulations (by wt.) can be:

| **Ingredient** | (about) |
|---|---|
| Vanillyl Butyl Ether | 0.03 to 0.20 g |
| Menthyl Lactate | 0.10 to 0.50 g |
| Menthol (crystal) | 0.03 to 0.15 g |
| Dimethicone | to 100 g |

To prepare the lubricant, for example, measure the right amount of vanillyl butyl ether in the empty beaker and then add in silicone oil. Stir with moderate speed until the mixture is homogenous. After that, add in the measured amount of menthyl lactate and menthol crystal (grind to smaller pieces for easy dissolving). Stir with moderate speed until the mixture is homogenous.

In embodiments of the two sensation-providing agents menthyl lactate and menthol in a tingling lubricant it can again be important to control the ratio of menthol to menthyl lactate to achieve the desired immediate then sustained effect, without creating an irritating sensation. For example, menthol can comprises about 10% to about 30% (wt.), such as about 15% to about 25%, of the total of the two cooling agents.

When VBE is incorporated together with the mixture of the cooling agents, it increases the warming and cooling sensation, which indirectly gives a unique tingling feeling.

In embodiments, about 99% or more of the lubricant by weight is the non-water soluble silicone oil. In embodiments, about 99.5% or more of the lubricant is the non-water soluble silicone oil.

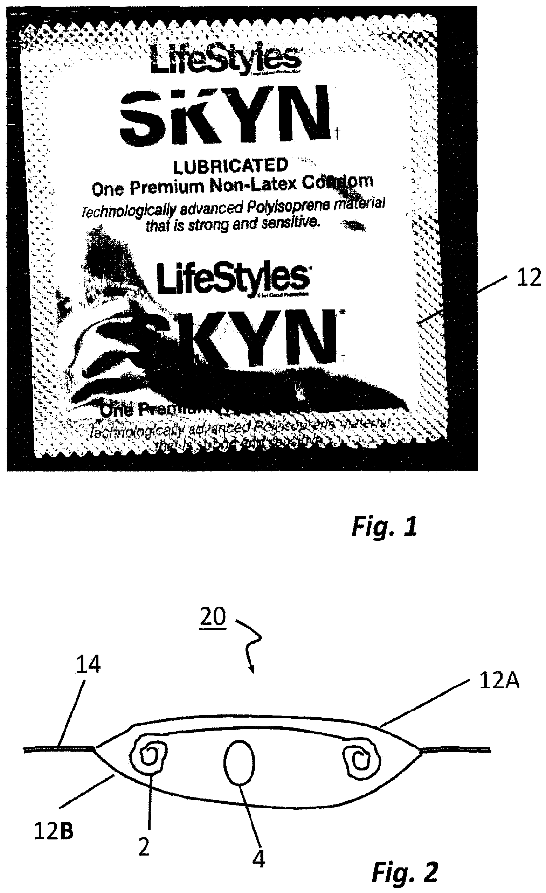

To package the condom dosed with lubricant, the lubricant composition (such as described above) is kept agitated (e.g. stirred) while metered amounts of lubricant are applied to the condom. Typically, the lubricant is applied to the rolled condom, such as in the cup formed by the rolling of the condom. If a male condom, for example, this is the opposite to the side on which the penis will be inserted. Typically, the Dimethicone (i.e., polydimethysiloxane) or other silicone oil will migrate over time and distribute itself over the entire exterior and interior surfaces of the condom. Fig. 2 illustrates a useful side from which to apply lubricant 4.

The agitation can be intermittent, so long as no substantial settling occurs. This means that the targeted concentrations of sensation-providing agents are found in the dosed lubricant (± about 1%).

In embodiments, the dosing of lubricant is from about 360 mg to about 480 mg per a standard size male condom, such as about 420 mg. For other sized condoms, the dosing can be adjusted with the surface area ratio.

The sensation-providing agents in question have polar groups such that their long term storage in silicone oil would not have been believed to be practical (though now their stable long term storage in silicone oil on polyisoprene has been established). The sensation-providing agents are:

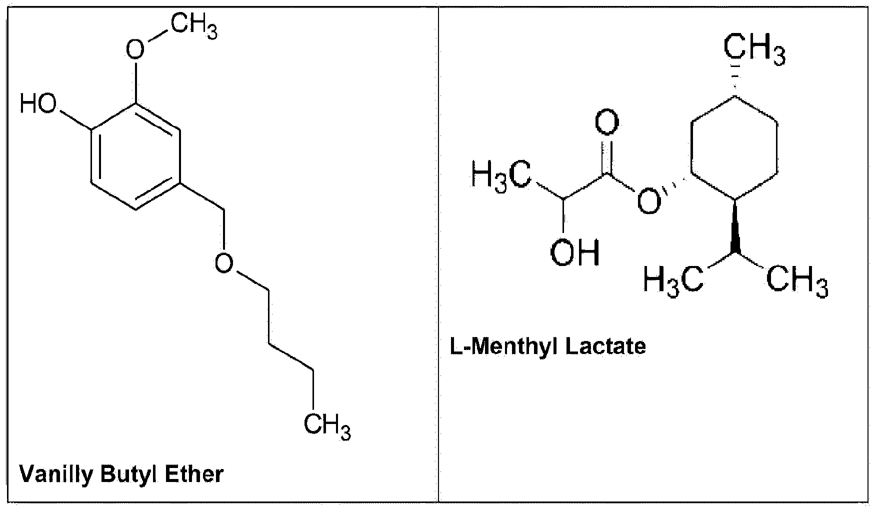

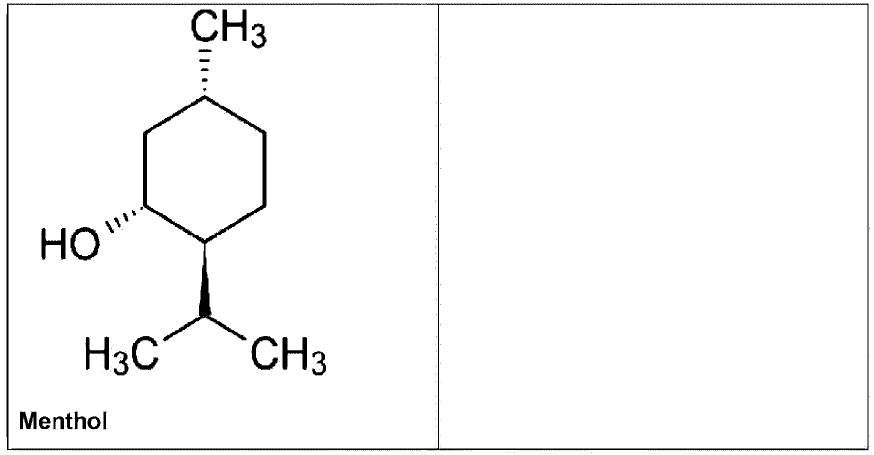

A solvent "consists essentially of non-water soluble silicone oil" when it is 99% or more non-water soluble silicone compounds (such as 99.5% or more, or 99.8% or more).

A lubricant is "essentially free of compounds that enhance the solubility of vanillyl butyl ether" in the non-water soluble silicone oil if the amounts of such compounds is less than enough required to increase vanillyl butyl ether solubility by 5%.

In embodiments, a lubricant is "essentially free of compounds that enhance the solubility of menthyl lactate" in the non-water soluble silicone oil, meaning that the amounts of such compounds is less than enough required to increase menthyl lactate solubility by 5%.

In embodiments, a lubricant is "essentially free of compounds that enhance the solubility of menthol" in the non-water soluble silicone oil, meaning that the amounts of such compounds is less than enough required to increase menthol solubility by 5%.

Generally the silicone oils relevant to this invention are polydimethylsiloxane or similar oils, with LogP of 10 or greater. Dimethiconol is believed to be tetramethyl-1,3-disiloxanediol, and believed to have a LogP of 2.69. A silicone oil "consists essentially of silicone compounds with LogP greater than 6" if the amount of silicone compounds with lower LogP is less than enough required to increase vanillyl butyl ether solubility by 5% (vs. a base solubility in the other silicone oil compounds).

A condom can be a male or female condom. In non-preferred embodiments, multiple condoms are in a single sealed package. The condom is a synthetic polyisoprene, meaning that the majority of repeating units by weight of the polymer component are isoprene. In embodiments, the synthetic polyisoprene is cis-1,4 poly-isoprene.

A package for a condom is one that, when sealed, isolates the condom from the environment, such that molecules within the package do not readily traverse the package boundaries, and oxygen does not do not readily traverse the package boundaries. The low level at which these molecules and oxygen do traverse is within the level accepted in the condom industry. One package used with a high level of such containment is where the packaging layers include a layer of aluminum foil. (The packaging and sealing is often termed "foiling," though metal foil is not always used.)

A "layer of aluminum" means that all sides of the packaging, except possibly the sealing edges (say when two pieces of packaging are sealed around the condom), include a layer of aluminum. If the aluminum comes from two pieces of packaging that is sealed, one could assert that there are two layers of aluminum - but that is not the meaning intended here.

Typically, a layer of aluminum as used to package condoms will be found in an aluminum composite laminate with a polymeric layer.

All ranges recited herein include ranges therebetween, and can be inclusive or exclusive of the endpoints. Optional included ranges are from integer values therebetween (or inclusive of one original endpoint), at the order of magnitude recited or the next smaller order of magnitude. For example, if the lower range value is 0.2, optional included endpoints can be 0.3, 0.4, ... 1.1, 1.2, and the like, as well as 1, 2, 3 and the like; if the higher range is 8, optional included endpoints can be 7, 6, and the like, as well as 7.9, 7.8, and the like. One-sided boundaries, such as 3 or more, similarly include consistent boundaries (or ranges) starting at integer values at the recited order of magnitude or one lower. For example, 3 or more includes 4 or more, or 3.1 or more.

A laminate is a bonding, fusing, adhesion, or the like between polymer layers, or between polymer and fabric layers, such that in the range of anticipated use the laminate is a unitary structure.

Where a sentence states that its subject is found in embodiments, or in certain embodiments, or in the like, it is applicable to any embodiment in which the subject matter can be logically applied.

This invention described herein is of a packaged lubricated condom and methods of forming the same. Although some embodiments have been discussed above, other implementations and applications are also within the scope of the following claims. Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the following claims.

## Claims

1. A packaged condom (20) comprising:
a sealed package;
within the package, a synthetic polyisoprene condom (2); and
within the package and substantially coating the condom, a lubricant (4) comprising solvent and one or more sensation-providing agents comprising one or more of vanillyl butyl ether, menthyl lactate, and menthol, optionally wherein the package comprises a layer of aluminum between the condom and an external environment, **characterized in that** the solvent comprises non-water soluble silicone oil and 99% or more of the lubricant (4) by weight is the non-water soluble silicone oil, wherein the solvent consists essentially of non-water soluble silicone oil, and said silicone oil consists essentially of silicone compounds with LogP greater than 6.

2. The packaged condom (20) of claim 1, wherein if the lubricant (4) comprises vanillyl butyl ether but neither menthyl lactate nor menthol in effective amounts, then (a) the solvent consists essentially of non-water soluble silicone oil, and (b) the lubricant (4) is essentially free of compounds that enhance the solubility of vanillyl butyl ether in the non-water soluble silicone oil.

3. The packaged condom (20) of claim 1, wherein sensation-providing agents comprise menthyl lactate or menthol.

4. The packaged condom (20) of claim 3, wherein sensation-providing agents comprise menthyl lactate and menthol, wherein the menthol comprises about 30% to about 45% (wt.) of the total of menthol and menthyl lactate.

5. The packaged condom (20) of one of claims 1 to 4, wherein sensation-providing agents comprise vanillyl butyl ether.

6. The packaged condom (20) of claim 1, wherein the sensation-providing agents comprise vanillyl butyl ether, menthyl lactate and menthol.

7. A method of packaging a condom (20), the method comprising the steps of:
providing an agitated lubricant (4) composition comprising solvent and one or more sensation-providing agents comprising one or more of vanillyl butyl ether, menthyl lactate, and menthol;
applying to a synthetic polyisoprene condom (2) a dose of agitated lubricant (4) composition; and
placing the condom (2) within a package and sealing it therein, optionally, wherein the package comprises a layer of aluminum between the condom and an external environment,
**characterized in that** the solvent comprises non-water soluble silicone oil and 99% or more of the lubricant (4) by weight is the non-water soluble silicone oil, wherein the solvent consists essentially of non-water soluble silicone oil, and that silicone oil consists essentially of silicone compounds with LogP greater than 6.

8. The method of claim 7, wherein if the lubricant (4) composition comprises vanillyl butyl ether but neither menthyl lactate nor menthol in effective amounts, then (a) the solvent consists essentially of non-water soluble silicone oil, and (b) the lubricant (4) is essentially free of compounds that enhance the solubility of vanillyl butyl ether in the non-water soluble silicone oil.

9. The method of claim 7, wherein sensation-providing agents comprise menthyl lactate or menthol.

10. The method of one of claims 7 to 9, wherein sensation-providing agents comprise vanillyl butyl ether.

11. The method of claim 7, wherein the sensation-providing agents comprise vanillyl butyl ether, menthyl lactate and menthol.

## Patentansprüche

1. Ein verpacktes Kondom (20), umfassend:
eine versiegelte Verpackung;
in der Verpackung ein synthetisches Polyisopren-Kondom (2); und
innerhalb der Verpackung und das Kondom im Wesentlichen bedeckend, ein Gleitmittel (4) umfassend ein Lösungsmittel und ein oder mehrere sensationserzeugende Mittel, umfassend eines oder mehrere von Vanillylbutylether, Menthyllactat und Menthol, wobei die Verpackung optional eine Aluminiumschicht zwischen dem Kondom und einer äußeren Umgebung umfasst,
**dadurch gekennzeichnet, dass** das Lösungsmittel nicht-wasserlösliches Silikonöl umfasst und 99 Gew.-% oder mehr des Gleitmittels (4) das nicht-wasserlösliches Silikonöl sind, wobei das Lösungsmittel im Wesentlichen aus nicht-wasserlöslichem Silikonöl besteht und das Silikonöl im Wesentlichen aus Silikonverbindungen mit einem LogP größer als 6 besteht.

2. Das verpackte Kondom (20) gemäß Anspruch 1, wobei, wenn das Gleitmittel (4) Vanillylbutylether, aber weder Menthyllactat noch Menthol in wirksamen Mengen umfasst, dann (a) das Lösungsmittel im Wesentlichen aus nicht-wasserlöslichem Silikonöl besteht, und (b) das Gleitmittel (4) im Wesentlichen frei von Verbindungen ist, die die Löslichkeit von Vanillylbutylether in dem nicht-wasserlöslichen Silikonöl verbessern.

3. Das verpackte Kondom (20) gemäß Anspruch 1, wobei die sensationserzeugenden Mittel Menthyllactat oder Menthol umfassen.

4. Das verpackte Kondom (20) gemäß Anspruch 3, wobei die sensationserzeugenden Mittel Menthyllactat und Menthol umfassen, wobei das Menthol etwa 30% bis etwa 45% (Gew.) der Gesamtmenge an Menthol und Mentyllactat umfasst.

5. Das verpackte Kondom (20) gemäß einem der Ansprüche 1 bis 4, wobei die sensationserzeugenden Mittel Vanillylbutylether umfassen.

6. Das verpackte Kondom (20) gemäß Anspruch 1, wobei die sensationserzeugenden Mittel Vanillylbutylether, Menthyllactat und Menthol umfassen.

7. Ein Verfahren zum Verpacken eines Kondoms (20), wobei das Verfahren die Schritte umfasst:
Bereitstellen einer gerührten Gleitmittel (4)-Zusammensetzung umfassend ein Lösungsmittel und ein oder mehrere sensationserzeugende Mittel umfassend eines oder mehrere von Vanillylbutylether, Menthyllactat und Menthol;
Auftragen einer Dosis einer gerührten Gleitmittel (4)-Zusammensetzung auf ein synthetisches Polyisopren-Kondom (2); und
Platzieren des Kondoms (2) in einer Verpackung und Versiegeln darin, wobei die Verpackung optional eine Aluminiumschicht zwischen dem Kondom und einer äußeren Umgebung umfasst,
**dadurch gekennzeichnet, dass** das Lösungsmittel ein nicht-wasserlösliches Silikonöl umfasst und 99 Gew.-% oder mehr des Gleitmittels (4) das nicht-wasserlösliche Silikonöl sind, wobei das Lösungsmittel im Wesentlichen aus dem nicht-wasserlöslichen Silikonöl besteht und das Silikonöl im Wesentlichen aus Silikonverbindungen mit einem LogP größer als 6 besteht.

8. Das Verfahren gemäß Anspruch 7, wobei, wenn die Gleitmittel (4)-Zusammensetzung Vanillylbutylether, aber weder Menthyllactat noch Menthol in wirksamen Mengen umfasst, dann (a) das Lösungsmittel im Wesentlichen aus nicht-wasserlöslichem Silikonöl besteht, und (b) das Gleitmittel (4) im Wesentlichen frei von Verbindungen ist, die die Löslichkeit von Vanillylbutylether in dem nicht-wasserlöslichen Silikonöl verbessern.

9. Das Verfahren gemäß Anspruch 7, wobei die sensationserzeugenden Mittel Menthyllactat oder Menthol umfassen.

10. Das Verfahren gemäß einem der Ansprüche 7 bis 9, wobei die sensationserzeugenden Mittel Vanillylbutylether umfassen.

11. Das Verfahren gemäß Anspruch 7, wobei die sensationserzeugenden Mittel Vanillylbutylether, Menthyllactat und Menthol umfassen.

## Revendications

1. Préservatif (20) emballé comprenant :
un emballage scellé ;
à l'intérieur de l'emballage, un préservatif (2) en polyisoprène synthétique ; et
à l'intérieur de l'emballage et recouvrant substantiellement le préservatif, un lubrifiant (4) comprenant un solvant et un ou plusieurs agents procurant des sensations comprenant un ou plusieurs éléments parmi l'éther de vanillyle butylique, le lactate de menthyle, et le menthol, facultativement sachant que l'emballage comprend une couche d'aluminium entre le préservatif et un environnement externe,
**caractérisé en ce que** le solvant comprend de l'huile de silicone non soluble dans l'eau et l'huile de silicone non soluble dans l'eau représente 99 % ou plus du lubrifiant (4) en poids, sachant que le solvant est composé essentiellement d'huile de silicone non soluble dans l'eau, et ladite huile de silicone est composée essentiellement de composés de silicone ayant un LogP supérieur à 6.

2. Le préservatif (20) emballé de la revendication 1, sachant que si le lubrifiant (4) comprend de l'éther de vanillyle butylique mais ni du lactate de menthyle ni du menthol en quantités effectives, alors (a) le solvant est composé essentiellement d'huile de silicone non soluble dans l'eau, et (b) le lubrifiant (4) est essentiellement exempt de composés qui augmentent la solubilité de l'éther de vanillyle butylique dans l'huile de silicone non soluble dans l'eau.

3. Le préservatif (20) emballé de la revendication 1, sachant que les agents procurant des sensations comprennent du lactate de menthyle ou du menthol.

4. Le préservatif (20) emballé de la revendication 3, sachant que les agents procurant des sensations comprennent du lactate de menthyle ou du menthol, sachant que le menthol comprend environ 30 % à environ 45 % (en poids) du total de menthol et de lactate de menthyle.

5. Le préservatif (20) emballé de l'une des revendications 1 à 4, sachant que les agents procurant des sensations comprennent de l'éther de vanillyle butylique.

6. Le préservatif (20) emballé de la revendication 1, sachant que les agents procurant des sensations comprennent de l'éther de vanillyle butylique, du lactate de menthyle et du menthol.

7. Procédé d'emballage d'un préservatif (20), le procédé comprenant les étapes de :
mise à disposition d'une composition de lubrifiant (4) agitée comprenant un solvant et un ou plusieurs agents procurant des sensations comprenant un ou plusieurs éléments parmi l'éther de vanillyle butylique, le lactate de menthyle, et le menthol ;
application, sur un préservatif (2) en polyisoprène synthétique, d'une dose de composition de lubrifiant (4) agitée ; et
placement du préservatif (2) dans un emballage et son scellage dans celui-ci, facultativement, sachant que l'emballage comprend une couche d'aluminium entre le préservatif et l'environnement externe,
**caractérisé en ce que** le solvant comprend de l'huile de silicone non soluble dans l'eau et l'huile de silicone non soluble dans l'eau représente 99 % ou plus du lubrifiant (4) en poids, sachant que le solvant est composé essentiellement d'huile de silicone non soluble dans l'eau, et l'huile de silicone est composée essentiellement de composés de silicone ayant un LogP supérieur à 6.

8. Le procédé de la revendication 7, sachant que si la composition de lubrifiant (4) comprend de l'éther de vanillyle butylique mais ni du lactate de menthyle ni du menthol en quantités effectives, alors (a) le solvant est composé essentiellement d'huile de silicone non soluble dans l'eau, et (b) le lubrifiant (4) est essentiellement exempt de composés qui augmentent la solubilité de l'éther de vanillyle butylique dans l'huile de silicone non soluble dans l'eau.

9. Le procédé de la revendication 7, sachant que les agents procurant des sensations comprennent du lactate de menthyle ou du menthol.

10. Le procédé de l'une des revendications 7 à 9, sachant que les agents procurant des sensations comprennent de l'éther de vanillyle butylique.

11. Le procédé de la revendication 7, sachant que les agents procurant des sensations comprennent de l'éther de vanillyle butylique, du lactate de menthyle et du menthol.
